# EUROPÄISCHE PATENTSCHRIFT

(11) **EP 0 883 808 B1**
(45) Veröffentlichungstag und Bekanntmachung des Hinweises auf die Patenterteilung: **26.04.2000**
(21) Anmeldenummer: 97904379.1
(22) Anmeldetag: 05.02.1997
(51) Int. Cl.: G01N 33/50, C12Q 1/02, C12N 5/06

(54) **VERFAHREN ZUR BESTIMMUNG DER PHOTOTOXIZITÄT UND/ODER PHOTOSENSIBILITÄT VON STOFFEN ODER STOFFGEMISCHEN SOWIE DESSEN VERWENDUNG**
PROCESS FOR DETERMINING THE PHOTOTOXICITY AND/OR PHOTOSENSITIVITY OF SUBSTANCES OR MIXTURES THEREOF, AND USE THEREOF
PROCEDE POUR DETERMINER LA PHOTOTOXICITE ET/OU LA PHOTOSENSIBILITE DE SUBSTANCES OU DE MELANGES DE SUBSTANCES, ET SON UTILISATION

(30) Priorität: 21.02.1996 DE 19606207
(43) Veröffentlichungstag der Anmeldung: 16.12.1998
(73) Patentinhaber: Neumann, Norbert J., 40229 Düsseldorf (DE)
(72) Erfinder: NEUMANN, Nobert, J., D-40229 Düsseldorf (DE); HÖLZLE, Erhard, D-21077 Hamburg (DE); ROSENBRUCH, Martin, D-40595 Düsseldorf (DE); PLEWIG, Gerd, D-80337 München (DE); LEHMANN, Percy, D-40591 Düsseldorf (DE)
(74) Vertreter: Sieckmann, Ralf, Dr.
(86) Internationale Anmeldenummer: EP9700508
(87) Internationale Veröffentlichungsnummer: WO9731266

(56) Entgegenhaltungen:
- WO-A-93/01272
- US-A- 5 405 369
- JOURNAL OF NATURAL PRODUCTS, Bd. 46, Nr. 5, September 1983, CINCINNATI, OHIO, Seiten 646-650, XP000197512 KAGAN ET AL.: "The effect of ultraviolet light on the toxicity of natural products toward the eggs of Drosophila melanogaster"
- PEDIATRIC RESEARCH, Bd. 32, Nr. 1, Juli 1992, BALTIMORE, MD, Seiten 23-26, XP000197364 GOLDBERG ET AL.: "Cardiac teratogenicity of dichloroethylene ...." in der Anmeldung erwähnt
- CANCER RESEARCH, Bd. 52, 15.Februar 1992, Seiten 924-930, XP000673041 ROBERTS ET AL.: "Role of neovasculature and vascular permeability ...." in der Anmeldung erwähnt
- BRITISH JOURNAL OF DERMATOLOGY, Bd. 136, Nr. 3, März 1997, OXFORD, Seiten 326-330, XP000197502 NEUMANN ET AL.: "Photo hen's egg test: a model for phototoxicity."

## Beschreibung

Die Erfindung betrifft ein Verfahren zur Bestimmung der Toxizität elektromagnetischer Strahlung, der Phototoxizität, und/oder Photosensibilität von Stoffen oder Stoffgemischen sowie dessen Verwendung.

Unter Phototoxizität im Sinne der vorliegenden Erfindung versteht man eine durch elektromagnetische Strahlung im Bereich von 1 mm bis 200 nm hervorgerufene Schädigung der Lokalverträglichkeit durch die Einwirkung von Infrarot-, sichtbarer und UV-Strahlung.

Insbesondere versteht man unter einer phototoxischen Reaktion eine durch die Bestrahlungsenergie und/oder sensibilisierende Substanzen, die exogen oder endogen in die Haut gelangen, zu einer Photodermatose führen. Hierbei absorbieren derartige phototoxische Substanzen nicht nur UV-Strahlung, sie können durch diese auch chemisch verändert werden. In Abhängigkeit von ihrer chemischen Struktur sind sie in verschiedenen Strahlungsbereichen absorptionsfähig. Es wird angenommen, daß zwischen der Menge an Strahlungsenergie und der von ihr getroffenen Substanz eine entsprechende Korrelation besteht. Wenig Energie und viel Substanz haben die gleiche Wirkung wie wenig Substanz und viel Strahlungsenergie. Bei einem maximalen Aufeinandertreffen beider Reaktionsparameter wird die phototoxische Reaktion besonders intensiv sein. Eine phototoxische Reaktion tritt in der Regel etwa 8 bis 12 Stunden nach der ersten Exposition auf. Mitunter kann dies auch früher der Fall sein. Übliche Symptome sind eine sehr starke Rötung verbunden mit Blasenbildung und einer später eintretenden postinflammatorischen Pigmentierung. Selbst ein Sonnenbrand kann als phototoxische Reaktion angesehen werden.

Unter einer photoallergischen Reaktion versteht man im Gegensatz zu einer phototoxischen Reaktion eine solche Reaktion die bereits auch bei leichterer Strahlung und wenig aktivierbarer Substanz auftritt. Diese ist auch nicht dosisabhängig. Der Mechanismus ist hier wie bei der Allergie, wobei die durch die Strahlung veränderte Fremdsubstanz zum Hapten werden kann, das sich mit dem Protein zu einem Allergen ausbildet. Die Erscheinung einer photoallergischen Reaktion manifestiert sich meist ekzematös. Sie tritt 48 bis 72 Stunden ab Beginn der Sonnenexposition auf. Entsprechend dem Entstehungsmechanismus einer Allergie tritt sie nie nach der ersten Exposition auf, da ja diese erst zur Bildung von Antikörpern führt. Photoallergische Reaktionen sind nicht auf die von der Strahlung exponierten Hautteile beschränkt. Sie treten im Sinne eines Streuphänomens auch an anderen, nicht bestrahlten Körperteilen auf. Das Aktionsspektrum ist gegenüber dem Absorptionsspektrum eher in den langwelligen Bereich verschoben. Besonders problematisch sind photoallergische Allergien als Reaktion auf chemisch verwandte Substanzen, wie beispielsweise Parastoffe. Die am Phenylring in Parastellung substituierte Amino-, Hydroxy- und Nitrogruppe ist das strukturtechnische Merkmal.

Es ist bereits bekannt, daß Hühnerembryos eingesetzt werden, um die toxikologischen Eigenschaften von Prüfsubstanzen zu untersuchen. Zumeist werden die Substanzproben zu diesem Zwecke über das extraembryonale Blutgefäßsystem dem Embryo zugeführt.

Zwei wissenschaftliche Veröffentlichungen aus der jüngsten Literatur seien stellvertretend für die geläufige Anwendung des Hühnerembryos als Modellsystem pharmakologischer (Roberts, W.G. und Hasan, T. CANCER RESEARCH, Band 52, 924 bis 930, 1992) oder toxikologischer Testreihen (Goldberg, S.J., Dawson, B.V., Johnson, P.D., Hoyme, H.E. und Ulreich, J.B., PEDIATRIC RESEARCH, Band 32, 23 bis 26, 1992) genannt.

In der Veröffentlichung von Roberts und Hasan werden die pharmakologischen/pharmakotopologischen Eigenschaften photodynamischer Agentien in proliferierenden vaskulären bzw. nonvaskulären Geweben des Hühnerembryos beschrieben. Die Hühnerembryogenese dient als Modellsystem für die Angiogenese in soliden Tumoren.

Die Veröffentlichung von Goldberg und Mitarbeitern beschreibt dagegen den Einsatz des Hühnerembryos als Methode, die Teratogenizität von Substanzen, insbesondere von Dichlorethylen, zu überprüfen. Durch die vorgenannten Veröffentlichungen wird allerdings weder vorbeschrieben noch nahegelegt, phototoxische und/oder photosensitive Substanzen am Hühnerembryo zu untersuchen.

Für die Untersuchung von potentiell phototoxischen bzw. photosensitiven Substanzen stehen bisher nur ein Tiermodell oder Zellsysteme zur Verfügung.

Beim Tiermodell handelt es sich um den "Draize-Test", der die Reizreaktion der phototoxischen Substanzen am Kaninchenauge erfaßt. Seine Anwendung wird nicht nur unter Kostengesichtspunkten, sondern insbesondere auch unter dem Gesichtspunkt des Tierschutzes begrenzt.

Die vorhandenen Zellsysteme, d.h. Bakterien-, Hefe- und andere Eukaryontenzellen, sind gleichfalls als Modelle für Phototoxizität unbefriedigend, weil sie die physiologischen Rahmenbedingungen des phototoxischen bzw. photosensitiven Substanzverhaltens nur unzureichend nachstellen können.

Der vorliegenden Erfindung liegt die Aufgabe zugrunde, ein technisch einfach gestaltetes Verfahren für den Test von potentiell phototoxischen Substanzen zu schaffen, das einerseits die physiologischen Gegebenheiten an der Haut berücksichtigt und andererseits auch zukünftigen gesetzgeberischen Vorhaben und ethischen Anforderungen genügt. Diese Aufgabe wird durch die Merkmale von Anspruch 1 gelöst.

Die vorliegende Erfindung betrifft somit einerseits ein Verfahren zur Bestimmung der Phototoxizität und/oder Photosensibilität von Stoffen oder Stoffgemischen, umfassend die Kontaktierung des chemischen Stoffes oder Stoffgemisches mit einem Embryo der Klasse Aves oder Geweben oder Gewebebestandteilen eines derartigen Embryos, wobei nach der Kontaktierung zusätzlich eine Behandlung mit einer elektromagnetischen Strahlung im Bereich von 1 mm bis 200 nm erfolgt sowie die nachfolgende Beurteilung der Embryo-, Gewebe- oder Zellpathologie, wobei vor der Kontaktierung Eier der Klasse Aves inkubiert werden, zu einem späteren Zeitpunkt nach der embryonalen Gastrulation ein Teil des Eiweißes über wenigstens eine Öffnung aus dem Ei entfernt wird, gegebenenfalls eine weitere Inkubation erfolgt und schließlich eine weitere Öffnung aus dem oberen Bereich der Eischale für die Belichtung vorgesehen wird.

Obgleich grundsätzlich ein beliebiges eierlegendes Wirbeltierembryo oder ein Gewebe oder ein Gewebebestandteil eines solchen Wirbeltierembryos eingesetzt werden kann, hat es sich aus praktischen Gründen als sinnvoll erwiesen, inkubierte Eier der Klasse Aves, also Vögel, einzusetzen. Beispielhaft seien hier genannt die entsprechenden Eier von Straußen, Nandus, Emus, Kiwis, Steißhühnern, Hühnervögeln, Hoazinen, Kampfwachteln, Tauben, Flughühnern, Rallen, Blatthühnchen, Kranichen, Trappen, Möwen, Wattvögel, Alken, Seetauchern, Lappentauchern, Pinguinen, Sturmvögeln, entenartigen Gänsevögeln, Ruderfüßlern, Schreitvögeln, Flamingos, Tagraubvögeln, Kuckucken, Turakus, Papageien, Racken, Eisvögeln, Bienenfressern, Widehopfen, Naßhornvögeln, Eulen, Ziegenmelkern, Seglern, Kolibries, Mausvögeln, Trogons, Pfefferfressern, Bartvögeln, Honiganzeigern, Spechten und Sperlingsvögeln. Besonders bevorzugt ist es, als inkubierte Vogeleier Eier der Gattung Galliformes, d.h. Hühnervögel, einzusetzen, worunter insbesondere Eier der Spezies Gallus bzw. auch Truthähne oder Puter fallen.

Die Dauer der Inkubation hängt üblicherweise von der Entwicklungszeit des jeweiligen Embryos ab.

Nach der Ausführungsform der vorliegenden Erfindung wird nach der ersten Inkubierung der Eier das Ei auch mit wenigstens einer Öffnung versehen. Diese Öffnung ist vorzugsweise so ausgestaltet, daß sie wenigstens eine Größe aufweist, daß durch die Öffnung ein Teil des Eiweißes entfernt werden kann. Dies geschieht vorzugsweise in der Art, daß mittels einer Absaugvorrichtung, vorzugsweise einer Pipette, wie beispielsweise einer Eppendorf-Pipette, ein Teil des Eiweißes, vorzugsweise 5 bis 10 ml des vorhandenen Eiweißes, entfernt wird.

Im Anschluß an die Absaugung erfolgt eine weitere Öffnung im oberen Bereich der Eierschale, um später eine Bestrahlung durchführen zu können. Dies geschieht im allgemeinen in der Weise, daß mit Hilfe einer mechanischen Vorrichtung eine weitere nun größere Öffnung in die Eierschale eingefügt wird, was beispielsweise durch eine scharfe Schneidender Fräßvorrichtung geschehen kann. Im Anschluß an die Anbringung der weiteren Öffnung im oberen Bereich der Eischale wird diese Öffnung wieder verschlossen und das Ei zurück in den Inkubator gegeben. Für das Verschließen der Öffnung verwendet man ein übliches Verschlußmittel, wobei es bevorzugt ist, beispielsweise eine Folie aus einem mettallenthaltenden oder aus einem organische Materialien enthaltenden Material zu verwenden. Beispielhaft seien hier Folien aus Kunststoff, Aluminium oder Verbundfolien genannt, ebenso wie entsprechende Wachspfropfen. Daraufhin wird gegebenenfalls wiederum eine Inkubation durchgeführt.

Im Anschluß an diese Inkubation werden vorzugsweise am vierten Tag der Bebrütung die zu testenden Substanzen aufgebracht, wobei es sich bei diesen chemischen Stoffen/Stoffgemischen um biologisch aktive Substanzen, beispielsweise Kosmetika, Pharmazeutika, Herbizide oder Insektizide, handeln kann. Weiterhin kann es sich bei diesen chemischen Stoffen/Stoffgemischen auch um Lichtschutzstoffe für technische Erzeugnisse sowie für kosmetische und pharmazeutische Zusammensetzungen handeln, welche auch unter dem Namen UV-Filter bekannt sind.

Die Behandlung des mit dem chemischen Stoffe oder Stoffgemisch behandelten Embryo der Klasse Aves oder Geweben oder Gewebsbestandteilen dieses Embryos erfolgt üblicherweise mit einer elektromagnetischen Bestrahlung im Bereich von 1 mm bis 200 nm, worunter man normalerweise den Bereich der Infrarotstrahlung, der Strahlung des sichtbaren Lichtes und der UV-Strahlung versteht.

Bevorzugt ist es allerdings, daß die Behandlung mit einer UV-Strahlung einer Wellenlänge kleiner als 400 nm erfolgt, insbesondere mit Wellenlängen zwischen 250 und 400 nm, d.h. meistens mit einer UV-A-Strahlung einer Wellenlänge zwischen 315 und 400 nm, alternativ einer UV-B-Strahlung einer Wellenlänge von 280 bis 315 nm.

Nach einer weiteren bevorzugten Ausführungsform erfolgt die vorbeschriebene Behandlung mit der elektromagnetischen Strahlung im Infrarotbereich in an sich bekannter Art mit Infrarotstrahlern, ebenso wie man im sichtbaren Bereich entsprechende Wellenlängen über Farbfilter oder ein Prisma einstellen kann. Für den Bereich der UV-Strahlung existieren zahlreiche Strahlungsquellen, wie beispielsweise Quecksilberdampfstrahler, Xenon-Höchstdruckstrahler, Natriumdampflampen, Wasserstoff- bzw. Deuteriumlampen sowie Niederdruckentladungsrohre mit Edelgasfüllungen. Darüber hinaus werden als UV-Strahler auch Wolframlampen bzw. Halogenlampen eingesetzt ebenso wie entsprechende Laser.

Sofern UV-Strahler eingesetzt werden, wird üblicherweise eine Bestrahlungsstärke von 1 mJ/cm² bis 100 J/cm² eingesetzt. Der obere Wert entspricht der maximalen UV-B-Dosis, der untere Wert der minimalen UV-A-Dosis. Bevorzugt ist der Einsatz von 5 bis 10 J/cm² UVA, insbesondere 5 J/cm².

Die erfindungsgemäße Kontaktierung des chemischen Stoffes mit den Geweben oder Gewebebestandteilen, vorzugsweise der chorionallantoischen Membran, insbesondere der Dottersackgefäßmembran, des Embryos der Klasse Aves erfolgt entweder sofort oder diese Kontaktierung kann auch bis zu 24 Stunden betragen.

Nach einer bevorzugten Ausführungsform erfolgt die Beurteilung der makro- und/oder mikroskopischen Embryonalpathologie wiederum entweder sofort oder innerhalb eines Zeitraums bis zu 24 Stunden, vorzugsweise aber nach 5 min bis zu 24 Stunden. Im Rahmen der vorgenannten Beurteilung der makro- und/oder mikroskopischen Embryonalpathologie wird entweder der Tod des Embryos, die Hämorrhagie, die Membranverfärbung oder die Gewebe- bzw. Zellpathologie überprüft.

Der vorliegenden Erfindung liegt weiterhin die Aufgabe zugrunde, ein Embryo der Klasse Aves, ein Gewebe oder ein Gewebebestandteil eines solchen Embryos zur Bestimmung der Phototoxizität und/oder Photosensibilität von chemischen Stoffen / Stoffgemischen gemäß vorbeschriebenen Verfahren einzusetzen.

Die vorliegende Erfindung wird nachfolgend durch Ausführungsbeispiele näher erläutert. Hierbei beziehen sich %-Angaben jeweils auf Gew.-%.

Zur Durchführung des erfindungsgemäßen Verfahrens wurden zwei Verbindungen mit wohlbekannten phototoxischen Eigenschaften eingesetzt, das 8-Methoxypsoralen und das Hematoporphyrin. Das Promethazin und das Ciprofloxazin wurden eingesetzt um die phototoxischen Eigenschaften mittels des erfindungsgemäßen Verfahrens zu beurteilen.

Hierzu mußte zunächst eine nicht-toxische Konzentration der vorgenannten Substanzen und eine nicht-toxische Dosis UVA-Strahlung experimentell definiert werden. Insofern wird das erfindungsgemäße Verfahren ähnlich durchgeführt wie der Hühnereitest selbst und dient zur Bestimmung der nicht-toxischen Wirkdosis einer Testsubstanz. In einem Vorversuch wurde herausgefunden, daß eine UVA-Strahlung von 5 J/cm² keine makroskopischen pathologischen Effekte auf den Eidottersack ausübt. Beim erfindungsgemäßen Verfahren wurde eine 10fach leichtere Konzentration eingesetzt, um eine nicht-toxische Wirkdosis der Testsubstanz an dem Eierdottersack anzuwenden und toxische Reaktionen sicher auszuschließen. Nur in einem Fall, d.h. in Gegenwart von Ciprofloxazin, wurde eine nicht-toxische Konzentration eingesetzt, die klinisch für intravenöse Injektionen eingesetzt wurde.

Die zweite Stufe des erfindungsgemäßen Verfahrens ist eine Bewertung im Sinne eines 2 x 2 faktoriellen Test Designs mit den Faktoren "Bestrahlung" und "Substanz Applikation" und den Stufen "ja" und "nein", wie aus der nachstehenden Übersicht ersichtlich.

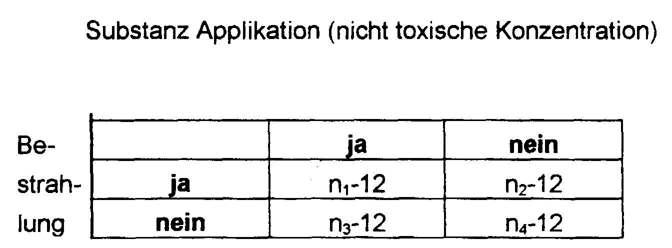

Innerhalb einer Dauer von 24 Stunden wurden folgende Parameter ausgewertet. Der Tod des Embryos, die halbquantitative Membranverfärbung und die Hämorrhagie. Dies wurde wie folgt durchgeführt. Befruchtete weiße Langhorneier (der Sorte Shaver Starcross 288A, Lohmann Tierzucht GmbH, Cuxhaven, Deutschland) wurden in einer horizontalen Position in einem üblichen Inkubator bei 37,5 °C und 65 % relativer Luftfeuchtigkeit inkubiert. Nach einer 3-tägigen Inkubation wurden alle Eier durchleuchtet, um solche auszuordnen, die defekt waren. Ohne die Membranschale zu verletzen, wurde ein Loch in die Schale gebohrt, durch die 5 ml Eiweiß abgezogen wurden, um das Embryo und den ihn umhüllenden Eidottersack zu erniedrigen. Daraufhin wurde ein 1,5 x 2,5 cm großes Fenster aus der Eischale herausgesägt. Die Eier wurden mit einem entsprechend groß geformten Wachsstück bedeckt und zurück in den Inkubator gegeben. An Tag 4 der Inkubation wurden nur solche Eier mit normal entwickelten Embryos und Blutgefäßsystemen zum Test eingesetzt.

Die folgenden Testsubstanzen wurden appliziert: 8-Methoxypsoralen (8-MOP) 10⁻³ molar in physiologischer Kochsalzlösung, Hematoporphyrin 10⁻⁵ molar ebenfalls in physiologischer Kochsalzlösung (0,9 %-ige Natriumchloridlösung), Promethazin 10⁻³ molar in physiologischer Kochsalzlösung und Ciprofloxazin 6,035 x 10⁻³ molar in physiologischer Kochsalzlösung. Für jede Testsubstanz wurde das 2 x 2 faktorielle Test Design verwendet. Jedesmal wurde zu einer Probe von 12 Eiern 500 µl der Testsubstanz mittels einer Eppendorf-Pipette appliziert und daraufhin mit einem 5 J/cm² UVA-Strahler (320-420 nm, Philips TL 09/40W, Hamburg, Deutschland) bestrahlt. Jeweils 12 Eier dienten als Vergleich ohne Substanz, denen 500 µl physiologische Kochsalzlösung bzw. 500 µl physiologische Kochsalzlösung in Verbindung mit einer 5 J/cm² UVA-Bestrahlung ausgesetzt wurden. Eine Bewertung erfolgte unmittelbar danach sowie 5, 15, 30 und 45 min bzw. 1, 2, 4, 6, 8, 10 und bis zu 24 Stunden nach der Bestrahlung. Während dieser Zeit wurde der Tod der Embryos und halb-quantitativ anhand einer 4-Punkt-Skala die Membranverfärbung und die Hämorrhagie bestimmt.

Hierbei wurde folgende Klassifikation eingesetzt:

| | |
|---|---|
| Stufe 0 | keine sichtbare Membranverfärbung/Hämorrhagie |
| Stufe 1 (leicht) | gerade sichtbare Membranverfärbung/Hämorrhagie |
| Stufe 2 (mittel) | sichtbare Membranverfärbung/Hämorrhagie, Strukturen sind teilweise verschwommen |
| Stufe 3 (schwer) | sichtbare Membranverfärbungen/Hämorrhagie, Strukturen sind völlig verschwommen. |

Die vorgenannten Veränderungen wurden mittels eines Makroskops Typ M 420 der Firma Leitz Meßtechnik GmbH, Wetzlar, Deutschland aufgezeichnet. Um die Folgen statistisch analysieren zu können, wurden nicht parametrische Tests eingesetzt. Für die Membranverfärbungsparameter und die Hämorrhagie wurde der Kontingenztafeltest für spezielle Klassen (eine Version des Kruskal-Wallis Testes, modifiziert für geordnete Kategorien) eingesetzt. Die Todesraten wurden mittels des Fisher Kontingenztafeltests analysiert. Folglich wurden für jede Verbindung drei statistische Tests berechnet. Unter Berücksichtigung der Anzahl der Tests und um die vermehrte Wahrscheinlichkeit, ein signifikantes Ergebnis zu finden, zu kompensieren, wurde der α-Level angepaßt (α*). Folglich wurde jeder der drei Einzeltests bei einem Signifikanzwert von α*=0,05/3, d.h. 0,0167, durchgeführt.

### ERGEBNISSE

### Hematoporphyrin

Bei Hematoporphyrin (HP) wurden beträchtliche morphologische Änderungen mit einem Maximum nach 12 Stunden beobachtet. Nach 24 Stunden zeigten
75,0 % (n=9) der Eidottersäcke eine schwere Verfärbung der Membran, 16,7 % (n=2) eine mittlere und
8,3 % (n=1) eine leichte Verfärbung der Membran bei Substanzen, die sowohl mit HP wie auch UVA-Strahlung behandelt worden waren. Im Gegensatz dazu zeigten die Kontrollmessungen nur leichte oder mittlere Membranverfärbungen (bei alleinigem Einsatz von HP
33,3 % (n=4) leichte und
25,0 % (n=3) mittlere Membranverfärbungen; bei der Kontrollgruppe, die nur mit physiologischer Kochsalzlösung und UV-A behandelt worden ist
50,0 % (n=6) leichte Membranverfärbungen und
25,0 % (n=3) mittlere Membranverfärbungen; und schließlich bei der nur mit physiologischer Kochsalzlösung behandelten Vergleichsgruppe waren 50,0 % (n=6) leicht und
16,7 % (n=2) der Membranen mäßig verfärbt). Der Kontingenztafeltest für geordnete Klassen zeigte ein signifikantes Ergebnis (p<0,00004).

Nach 24 Stunden zeigten
50,0 % (n=6) der Eidottersäcke (Dottersackgefäßmembranen) eine mittlere und
50,0 % (n=6) eine leichte Hämorrhagie, bei der sowohl mit HP wie auch UV-A behandelten Gruppe. Im Gegensatz dazu zeigten die Vergleichsproben nur eine leichte Hämorrhagie (HP selbst
33,3 % (n=4) einer leichten Hämorrhagie, bei physiologischer Kochsalzlösung und UV-Bestrahlung
25,0 % (n=3) einer leichten Hämorrhagie; und bei physiologischer Kochsalzlösung allein
16,7 % (n=2) einer leichten Hämorrhagie). Der Kontingenztafeltest für geordnete Klassen zeigte ein signifikantes Ergebnis (p<0,00001).

Weiter wurde gefunden, daß in
41,7 % (n=5) der HP/UVA-Gruppe es zu einem Tod der Embryos kam. Im Gegensatz dazu wurde bei keiner der Vergleichsproben ein Tod der Embryos festgestellt. Fisher's Kontingenztafeltest zeigte ein signifikantes Ergebnis (p<0,0094).

### 8-Methoxypsoralen

Die wesentlichen morphologischen Veränderungen wurden nach einem Maximum von 12 Stunden beobachtet. Nach 24 Stunden zeigten
16,7 % (n=2) der Eidottersäcke schwere,
75,0 % (n=9) mittlere und
8,3 % (n=1) leichte Membranverfärbungen in der 8-MOP/UVA-Gruppe.
Die Vergleichsproben zeigten nur leichte bis mittlere Membranverfärbungen (MOP selbst
58,3 % (n=7) leichte und
33,3 % (n=4) mittlere Membranverfärbungen; bei der Gruppe, mit physiologischer Kochsalzlösung und UVA wurden bei
50,0 % (n=6) leicht verfärbte Membranen gefunden, bei der nur mit physiologischer Kochsalzlösung behandelten Vergleichsprobe fand man 41,7 % (n=5) leichte Membranverfärbung und
16,7 % (n=2) mittlere Membranverfärbung). Der Kontingenztafeltest für geordnete Klassen zeigte ein signifikantes Ergebnis (p<0,00002).

Nach 24 Stunden fand man bei
91,7 % (n=11) der Eidottersäcke eine schwere und
8,3 % (n=1) einer mittleren Hämorrhagie bei der 8-MOP/UVA-Gruppe. Die Vergleichsproben zeigten eine nur leichte Hämorrhagie (8-MOP selbst
16,7 % (n=2) leichte Hämorrhagie; bei der Gruppe, die mit physiologischer Kochsalzlösung und UVA gehandelt worden ist,
8,3 % (n=1) leichte Hämorrhagie; bei der nur mit physiologischer Kochsalzlösung behandelten Gruppe
8,3 % (n=1) leichte Hämorrhagie). Der Kontingenztafeltest für geordnete Klassen zeigte ein signifikantes Ergebnis (p<0,00000).

Für die 8-MOP/UVA-Gruppe wurde eine Letalität der Embryos von 100 % (n=12) festgestellt. Im Gegensatz dazu wurde bei den Vergleichsproben nie ein Tod der Embryos festgestellt. Fisher's Kontingenztafeltest zeigte ein signifikantes Resultat (p<0,00000).

### Promethazin

Eine bedeutende Schädigung der Dottersackgefäßmembran mit einer Spitze 12 Stunden nach der Bestrahlung wurde nur in Bezug auf Promethazin (PMZ) in Kombination mit UVA-Strahlung festgestellt. Nach 24 Stunden zeigten
66,7 % (n=8) der Eidottersäcke eine schwere,
8,3 % (n=1) eine mittlere und
25,0 % (n=3) eine leichte Membranverfärbung in der PMZ/UVA-Gruppe. Die Kontrollproben zeigten nur eine leichte oder mittlere Membranverfärbung (PMZ selbst
50,0 % (n=6) leichte Membranverfärbung; die mit physiologischer Kochsalzlösung und UVA behandelte Gruppe
58,3 % (n=7) leichte Membranverfärbungen; und die ausschließlich mit physiologischer Kochsalzlösung behandelte Gruppe zeigte
58,3 % (n=7) leichte Membranverfärbungen und
8,3 % (n=1) mittlere Membranverfärbungen). Der Kontingenztafeltest für geordnete Klassen zeigte ein signifikantes Ergebnis (p<0,00005).

Nach 24 Stunden fand man bei
41,7 % (n=5) der Dottersackgefäßmembran eine schwere, bei 41,7 % (n=5) eine mittlere und bei
8,3 % (n=1) eine leichte Hämorrhagie, in der PMZ/UBA-Gruppe. Die Kontrollversuche zeigten nur eine leichte Hämorrhagie (PMZ selbst
8,3 % (n=1) leichte Hämorrhagie; bei Einsatz einer physiologischen Kochsalzlösung und einer UVA-Bestrahlung
8,3 % (n=1) einer leichten Hämorrhagie; und bei physiologischer Kochsalzlösung selbst bei
8,3 % (n=1) einer leichten Hämorrhagie). Der Kontingenztafeltest für geordnete Kategorien zeigte ein signifikantes Ergebnis (p<0,00000).

Nach 24 Stunden waren
100 % der Embryos in der PMZ/UVA-Gruppe gestorben. Nur zwei tote Embryos fand man in den Vergleichsgruppen (nämlich bei der Gruppe mit physiologischer Kochsalzlösung und UVA
8,3 % (n=1) sowie bei physiologischer Kochsalzlösung selbst
8,3 % (n=1)). Fisher's Kontingenztafeltest zeigte ein signifikantes Ergebnis (p<0,00000).

### Ciprofloxazin

Eine bedeutende Schädigung der Dottersackgefäßmembran wurde nur bei einer Kombination von Ciprofloxazin (CF) mit UVA gefunden. Nach 24 Stunden waren
91, 7 % (n=11) der Dottersackgefäßmembranen schwer und
8,3 % (n=1) der Membranen mäßig in der CF/UVA-Gruppe verfärbt. Dem gegenüber zeigten die Vergleichsproben leichte und mittlere Membranverfärbungen (CF selbst
75,0 % (n=9) leichte Membranverfärbungen und
25,0 % (m=3) mittlere Membranverfärbungen; bei der Gruppe, die mit physiologischer Kochsalzlösung und UVA behandelt wurde, fand man 66,7 % (n=8) leichte Membranverfärbung und
8,3 % (n=1) mittlere Membranverfärbung. Bei der ausschließlich mit physiologischer Kochsalzlösung fand man
58,3 % (n=7) leichte Membranverfärbungen und
8,3 % (n=1) mittlere Membranverfärbungen.). Der Kontingenztafeltest für geordnete Klassen zeigte ein signifikantes Ergebnis (p<0,00000).

Nach 24 Stunden zeigten
50,0 % (n=6) der Dottersackgefäßmembranen eine leichte Hämorrhagie und
16,7 % (n=2) eine mittlere Hämorrhagie in der CF/UVA-Gruppe. Die Vergleichsproben zeigten nur eine leichte Hämorrhagie (CF selbst
41,7 % (n=5) leichte Hämorrhagie. In der mit physiologischer Kochsalzlösung und UVA behandelten Gruppe fand man
25,0 % (n=3) leichte Hämorrhagie und in der ausschließlich mit physiologischer Kochsalzlösung behandelten Gruppe
33,3 % (n=4) leichte Hämorrhagie.). Diese Gruppen unterschieden sich nicht deutlich untereinander. Keines der Embryos starb in irgendeiner der Gruppen.

## Patentansprüche

1. Verfahren zur Bestimmung der Phototoxizität, und/oder Photosensibilität von Stoffen oder Stoffgemischen, umfassend die Kontaktierung des chemischen Stoffes oder Stoffgemisches mit einem Embryo der Klasse Aves oder Geweben oder Gewebebestandteilen eines derartigen Embryos, wobei nach der Kontaktierung zusätzlich eine Behandlung mit einer elektromagnetischen Strahlung im Bereich von 1 mm bis 200 nm erfolgt sowie die nachfolgende Beurteilung der Embryo-, Gewebe- oder Zellpathologie, wobei vor der Kontaktierung Eier der Klasse Aves inkubiert werden, zu einem späteren Zeitpunkt nach der embryonalen Gastrulation ein Teil des Eiweißes über wenigstens eine Öffnung aus dem Ei entfernt wird, gegebenenfalls eine weitere Inkubation erfolgt und schließlich eine weitere Öffnung aus dem oberen Bereich der Eischale für die Belichtung vorgesehen wird.

2. Verfahren nach Anspruch 1 **dadurch gekennzeichnet**, daß die chemischen Stoffe/Stoffgemische biologisch aktive Substanzen, insbesondere Pharmazeutika, Herbizide oder Insektizide umfassen oder Lichtschutzstoffe für technische oder biologisch aktive Substanzen sind.

3. Verfahren nach einem der vorstehenden Ansprüche, **dadurch gekennzeichnet**, daß die Behandlung mit einer UV-Strahlung einer Wellenlänge < 400 nm, vorzugsweise im Bereich zwischen 400 nm und 250 nm erfolgt.

4. Verfahren nach einem der vorstehenden Ansprüche, **dadurch gekennzeichnet**, daß die UV-Strahlung mit einer Bestrahlungsstärke von 1 mJ/cm² bis 100 J/cm², vorzugsweise 1 bis 20 J/cm² UVA, insbesondere 5 bis 10 J/cm² UVA erfolgt.

5. Verfahren nach einem der vorstehenden Ansprüche, **dadurch gekennzeichnet**, daß die Kontaktierung des Stoffgemisches für eine Zeit von 10 sec bis 24 Stunden mit Geweben oder Gewebebestandteilen, vorzugsweise der Dottersackgefäßmembran oder der chorionallantoischen Membran des Embryos erfolgt.

6. Verfahren nach einem der vorstehenden Ansprüche, **dadurch gekennzeichnet**, daß die Beurteilung der makro- und/oder mikroskopischen Embryonalpathologie innerhalb einer Zeit von bis zu 96 Stunden, vorzugsweise zwischen 5 min bis 24 Stunden erfolgt.

7. Verfahren nach einem der vorstehenden Ansprüche, **dadurch gekennzeichnet**, daß der Tod des Embryos, Hämorrhagie, Membranverfärbung oder die Gewebe- bzw. die Zellpathologie beurteilt werden.

8. Verfahren nach Anspruch 1, **dadurch gekennzeichnet**, daß Eier der Gattung Galliformes insbesondere aber Eier der Gattung Gallus oder Truthahn eingesetzt werden.

9. Verwendung eines Embryos der Klasse Aves oder Geweben oder Gewebebestandteilen eines solchen Embryos zur Bestimmung der Phototoxizität, und/oder Photosensibilität von chemischen Stoffen/Stoffgemischen gemäß irgendeinem der Ansprüche 1 bis 8.

## Claims

1. Process for determining the phototoxicity and / or photosensitivity of substance mixtures, involving putting the chemical substance or substance mixture into contact with embryos of the class *Aves* or tissues or tissue components of such an embryo, whereby, following the contact, there is an additional treatment with electromagnetic radiation ranging from 1 mm to 200 nm, followed by evaluation of the pathology of the embryo, tissue, or cell whereby prior to the contact, eggs of the *Aves* class are incubated and, at a later point in time after the embryonic gastrulation, some of the egg white is removed from the egg via at least one opening and, if applicable, further incubation takes place and then another opening is provided in the upper section of the egg shell for purposes of the radiation.

2. Process according to Claim 1, **characterised In that** the chemical substance/substance mixture encompass biologically active substances, particularly pharmaceuticals, herbicides or insecticides or light-protection agents for technical or biologically active substances.

3. Process according to any of the preceding Claims, **characterised In that** the treatment is done employing UV radiation having a wavelength of < 400 nm, preferably within the range from 400 nm to 250 nm.

4. Process according to any of the preceding Claims, **characterised in that** the UV-radiation is done with a radiation level ranging from 1 mJ/cm² to 100 J/cm², preferably from 1 to 20 J/cm² of UV-A, especially 5 to 10 J/cm² of UV-A.

5. Process according to one of the preceding Claims, **characterised In that** the contacting of the substance mixture with tissues or tissue components, preferably the yolk vessel membrane or the chorioallantoic membrane of the embryo, is done for a period of time ranging from 10 seconds to 24 hours.

6. Process according to any of the preceding Claims, **characterised in that** the macroscopic and / or microscopic embryonic pathology is evaluated within period of time up to 96 hours, preferably between 5 minutes and 24 hours.

7. Process according to one of the preceding Claims, **characterised in that** the death of the embryo, hemorrhaging, membrane discoloration or the tissue and / or cell pathology are evaluated.

8. Process according to Claim 1, **characterised in that** eggs of the *Galliformes* order, especially chickens or turkey are used.

9. Use of an embryo of the *Aves* class or tissues or tissue components of such an embryo for purposes of determining the phototoxicity and / or photosensitivity of biological substances / substance mixtures in accordance with any of Claims 1 to 8.

## Revendications

1. Procédé pour déterminer la phototoxicité et / ou la photosensibilité des substances ou mélanges de substances qui comprend le contact des substances ou mélanges de substances chimiques avec un embryon de la classe Aves ou des tissus ou éléments de tissus d'un tel embryon et, après le contact, un traitement supplémentaire avec une radiation électromagnétique dans la région de 1 mm à 200 nm et ensuite une évaluation de la pathologie de l'embryon, du tissu ou de la cellule et ,avant le contact, une incubation des oeufs de la classe Aves et ,quelque temps après la gastrulation embroynnaire un prélèvement d'une part du blanc d'oeuf par au moins une ouverture et, le cas échéant, une incubation de plus et finalement une ouverture de plus dans la partie supérieure de la coquille d'oeuf pour la radiation.

2. Procédé selon revendication 1, **caractérisé en ce que** les substances et les mélanges de substances chimiques comprennent des substances biologiquement actives , notamment des produits pharmaceutiques, herbicides ou insecticides ou des substances protectrices contre la lumière pour des substances techniques ou des substances biologiquement active.

3. Procédé selon l'une des revendications précédentes, **caractérisé en ce que** le traitement est effectué avec une radiation U.V. avec une longueur d'onde de < 400 nm, de préférence dans la région de 400 nm à 250 nm.

4. Procédé selon l'une des revendications précédentes, **caractérisé en ce que** la radiation U.V. est réalisée avec une intensité dans la région de 1 mJ/cm² à 100 J/cm², de préférence de 1 à 20 J/cm² U.V.-A, notamment de 5 à 10 J/cm² U.V.-A.

5. Procédé selon l'une des revendications précédentes, **caractérisé en ce que** le contact des mélanges de substance s'effectue pendant une période de 10 sec à 24 heures avec des tissus ou éléments de tissu, de préférence la membrane vasculaire du sac vittelin ou la membrane chorioallantoic de l'embryon.

6. Procédé selon l'une des revendications précédentes, **caractérisé en ce que** l'évaluation de la pathologie macroscopique et / ou microscopique de l'embryon est effectuée dans une période de 96 heures au maximum, de préférence entre 5 minutes et 24 heures.

7. Procédé selon l'une des revendications précédentes, **caractérisé en ce que** la mort de l'embryon, l'hémorragie, l'altération de couleur de la membrane ou la pathologie du tissu ou de la cellule sont évaluées.

8. Procédé selon revendication 1, **caractérisé en ce que** les oeufs du genre Galliformes , mais notamment les oeufs du genre Gallus ou dindon sont utilisés.

9. L'utilisation d'un embryon de la classe Aves ou des tissus ou éléments de tissus d'un tel embryon pour déterminer la phototoxicité et / ou photosensibilité des substances ou des mélanges de substances chimiques selon l'une des revendications 1 à 8 n'importe quelle .
